# EUROPEAN PATENT APPLICATION

(11) **EP 3 109 689 A1**
(43) Date of publication of application: **28.12.2016**
(21) Application number: 15173068.6
(22) Date of filing: 22.06.2015
(51) Int. Cl.: G02B 27/01, G06F 1/32, A61B 5/11

(54) **TRANSITION FROM A DISPLAY POWER MODE TO A DIFFERENT DISPLAY POWER MODE**

(71) Applicant: Nokia Technologies Oy, 02610 Espoo (FI)
(72) Inventor: ERONEN, Antti, 33820 Tampere (FI); LEPPÄNEN, Jussi, 33580 Tampere (FI); LEHTINIEMI, Arto, 33880 Lempäälä (FI); ROIMELA, Kimmo, 33710 Tampere (FI)
(74) Representative: Nokia Corporation

(57) **Abstract**

A method comprising causing rendering of information on a display, comprised by a head mounted display, in a non-blink display power mode, receiving ocular sensor information from at least one ocular sensor, the ocular sensor information indicating eye activity of a user of the head mounted display, determining a user blink initiation of the user based, at least in part, on the ocular sensor information, transitioning from the non-blink display power mode to a blink display power mode in response to the determination of the user blink initiation, determining a user blink termination of the user based, at least in part, on the ocular sensor information, and transitioning from the blink display power mode to the non-blink display power mode in response to the determination of the user blink termination is disclosed. The ocular sensor may comprise electrooculography information.

## Description

### TECHNICAL FIELD

The present application relates generally to transition from a display power mode to a different display power mode.

### BACKGROUND

In modern times, electronic apparatus are often powered by a power source, such as electrical batteries, electrical cells, and/or the like that have a limited power storage capacity. Such electronic devices may operate for extended periods without having their power sources recharged. As such, it may be desirable to configure an apparatus such that the apparatus utilizes the power source in a manner that conserves the limited power of the power source.

### SUMMARY

Various aspects of example embodiments are set out in the summary, the drawings, the detailed description, and the claims.

One or more example embodiments may provide an apparatus, a computer readable medium, a non-transitory computer readable medium, a computer program product, and/or a method for causing rendering of information on a display, comprised by a head mounted display, in a non-blink display power mode, receiving ocular sensor information from at least one ocular sensor, the ocular sensor information indicating eye activity of a user of the head mounted display, determining a user blink initiation of the user based, at least in part, on the ocular sensor information, transitioning from the non-blink display power mode to a blink display power mode in response to the determination of the user blink initiation, determining a user blink termination of the user based, at least in part, on the ocular sensor information, and transitioning from the blink display power mode to the non-blink display power mode in response to the determination of the user blink termination.

One or more example embodiments may provide an apparatus, a computer readable medium, a computer program product, and/or a non-transitory computer readable medium having means for causing rendering of information on a display, comprised by a head mounted display, in a non-blink display power mode, means for receiving ocular sensor information from at least one ocular sensor, the ocular sensor information indicating eye activity of a user of the head mounted display, means for determining a user blink initiation of the user based, at least in part, on the ocular sensor information, means for transitioning from the non-blink display power mode to a blink display power mode in response to the determination of the user blink initiation, means for determining a user blink termination of the user based, at least in part, on the ocular sensor information, and means for transitioning from the blink display power mode to the non-blink display power mode in response to the determination of the user blink termination.

An apparatus comprising at least one processor and at least one memory, the memory comprising machine-readable instructions, that when executed cause the apparatus to perform causation of rendering of information on a display, comprised by a head mounted display, in a non-blink display power mode, receipt of ocular sensor information from at least one ocular sensor, the ocular sensor information indicating eye activity of a user of the head mounted display, determination of a user blink initiation of the user based, at least in part, on the ocular sensor information, transition from the non-blink display power mode to a blink display power mode in response to the determination of the user blink initiation, determination of a user blink termination of the user based, at least in part, on the ocular sensor information, and transitioning from the blink display power mode to the non-blink display power mode in response to the determination of the user blink termination.

In at least one example embodiment, the blink display power mode is a power mode of the head mounted display that consumes less power than the non-blink display power mode.

In at least one example embodiment, power consumed by the head mounted display in the non-blink display power mode is greater than power consumed by the head mounted display in the blink display power mode.

In at least one example embodiment, power consumed by the head mounted display for purposes of display of information in the non-blink display power mode is greater than power consumed by the head mounted display for purposes of display of information in the blink display power mode.

In at least one example embodiment, illumination of the display in the non-blink display power mode is greater than illumination of the display in the blink display power mode.

In at least one example embodiment, transitioning from the non-blink display power mode to the blink display power mode comprises reduction of illumination of the display.

In at least one example embodiment, reduction of illumination of the display comprises termination of illumination of the display.

In at least one example embodiment, transitioning from the blink display power mode to the non-blink display power mode comprises increase of illumination of the display.

In at least one example embodiment, increase of illumination of the display comprises initiation of illumination of the display.

In at least one example embodiment, information rendered by the display in the non-blink display power mode is greater than information rendered by the display in the blink display power mode.

In at least one example embodiment, transitioning from the non-blink display power mode to the blink display power mode comprises reduction of information rendered by the display.

In at least one example embodiment, reduction of information rendered by the display comprises disablement of the display.

In at least one example embodiment, transitioning from the blink display power mode to the non-blink display power mode comprises increase of information rendered by the display.

In at least one example embodiment, increase of information rendered by the display comprises enablement of the display.

In at least one example embodiment, power consumed by graphics processing hardware in the non-blink display power mode is greater than power consumed by graphics processing hardware in the blink display power mode.

In at least one example embodiment, transitioning from the non-blink display power mode to the blink display power mode comprises reduction of power consumed by graphics processing hardware.

In at least one example embodiment, reduction of power consumed by graphics processing hardware comprises enablement of a low power mode of the graphics processing hardware.

In at least one example embodiment, reduction of power consumed by graphics processing hardware comprises disablement of the graphics processing hardware.

In at least one example embodiment, transitioning from the blink display power mode to the non-blink display power mode comprises increase of power consumed by graphics processing hardware.

In at least one example embodiment, increase of power consumed by graphics processing hardware comprises disablement of a low power mode of the graphics processing hardware.

In at least one example embodiment, increase of power consumed by graphics processing hardware comprises enablement of the graphics processing hardware.

In at least one example embodiment, power consumed by non-video hardware in the non-blink display power mode is equivalent to power consumed by non-video hardware in the blink display power mode.

In at least one example embodiment, transitioning from the non-blink display power mode to the blink display power mode avoids change of power consumed by non-video hardware.

In at least one example embodiment, transitioning from the blink display power mode to the non-blink display power mode avoids change of power consumed by non-video hardware.

One or more example embodiments further perform receipt of other ocular sensor information from the at least one ocular sensor, the other ocular sensor information indicating other eye activity of the user.

In at least one example embodiment, the determination of the user blink termination is based, at least in part, on the other ocular sensor information.

One or more example embodiments further perform determination of an average blink duration.

In at least one example embodiment, the determination of the user blink termination is based, at least in part, on elapse of the average blink duration subsequent to the user blink initiation.

In at least one example embodiment, the ocular sensor comprises at least one camera module, and the ocular sensor information comprises visual information indicative of an eye of the user.

In at least one example embodiment, the determination of a user blink initiation comprises determination of visual information indicative of a transition from the eye being open to the eye being closed.

In at least one example embodiment, the determination of a user blink termination comprises determination of visual information indicative of a transition from the eye being closed to the eye being open.

In at least one example embodiment, the ocular sensor comprises a plurality of electrodes, and the ocular sensor information comprises electrooculography information indicative of an eye of the user.

In at least one example embodiment, the head mounted display is configured to have at least one of the plurality of electrodes contact a front of the user's nose bridge.

In at least one example embodiment, the head mounted display is configured in the form of glasses, and at least one the plurality of electrodes is disposed on a middle of a bridge of the glasses to contact the user's nose

In at least one example embodiment, the head mounted display is configured to have at least one of the plurality of electrodes contact a right side of the user's nose bridge.

In at least one example embodiment, the head mounted display is configured to have at least one of the plurality of electrodes contact a left side of the user's nose bridge.

In at least one example embodiment, the head mounted display is configured in the form of glasses, and at least one the plurality of electrodes is disposed on a right side of a bridge of the glasses to contact the user's nose.

In at least one example embodiment, the head mounted display is configured in the form of glasses, and at least one the plurality of electrodes is disposed on a left side of a bridge of the glasses to contact the user's nose.

In at least one example embodiment, the determination of a user blink initiation comprises determination that the electrooculography information indicates a transition from the eye being open to the eye being closed.

In at least one example embodiment, the determination that the electrooculography information indicates a transition from the eye being open to the eye being closed comprises determination that a vertical component of the electrooculography information has increased to exceed a blink threshold value.

In at least one example embodiment, the blink threshold value is a predetermined value.

One or more example embodiments further perform determination of a running average of the electrooculography information over a predetermined duration, and determination of the blink threshold value to be proportional to the running average.

One or more example embodiments further perform determination of a standard deviation of the electrooculography information over the predetermined duration.

In at least one example embodiment, the determination of the blink threshold value is performed such that the blink threshold value is proportional to the standard deviation.

One or more example embodiments further perform determination of a battery power level of the head mounted display, and determination of the blink threshold value to be proportional to the battery power level.

In at least one example embodiment, the determination of a user blink termination comprises determination that the electrooculography information indicates a transition from the eye being closed to the eye being open.

In at least one example embodiment, the determination that the electrooculography information indicates a transition from the eye being closed to the eye being open comprises determination that a vertical component of the electrooculography information has decreased to become within a blink threshold value.

### BRIEF DESCRIPTION OF THE DRAWINGS

For a more complete understanding of one or more example embodiments, reference is now made to the following descriptions taken in connection with the accompanying drawings in which:
FIG. 1 is a block diagram showing an apparatus according to at least one example embodiment;
FIGS. 2A-2C are diagrams illustrating head mounted displays according to at least one example embodiment,
FIG. 3 is a diagram illustrating a video pipeline according to at least one example embodiment;
FIG. 4 is a diagram illustrating ocular sensors according to at least one example embodiment,
FIG. 5 is a diagram illustrating ocular sensors according to at least one example embodiment,
FIG. 6 is a diagram illustrating ocular sensor information according to at least one example embodiment,
FIG. 7 is a flow diagram illustrating activities associated with transition from a display power mode to a different display power mode according to at least one example embodiment,
FIG. 8 is a flow diagram illustrating activities associated with receipt of electrooculography information according to at least one example embodiment,
FIG. 9 is a flow diagram illustrating activities associated with determination of a standard deviation of electrooculography information according to at least one example embodiment; and
FIG. 10 is a flow diagram illustrating activities associated with determination of a battery power level according to at least one example embodiment.

### DETAILED DESCRIPTION OF THE DRAWINGS

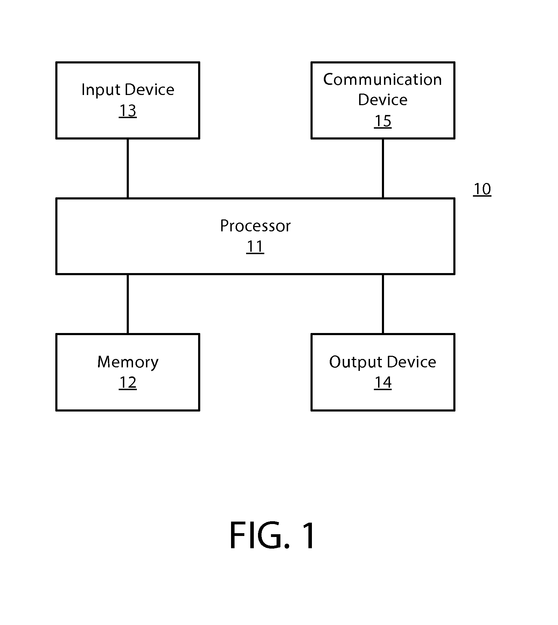

Various example embodiments and some of their potential advantages are understood by referring to FIGS. 1 through 10 of the drawings.

Some example embodiments will now further be described hereinafter with reference to the accompanying drawings, in which some, but not all, example embodiments are shown. One or more example embodiments may be embodied in many different forms and the claims should not be construed as being strictly limited to the example embodiments set forth herein; rather, these example embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with one or more example embodiments. Thus, use of any such terms should not be taken to limit the spirit and scope of example embodiments.

Additionally, as used herein, the term 'circuitry' refers to (a) hardware-only circuit implementations (e.g., implementations in analog circuitry, digital circuitry and/or any combination thereof); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that utilize software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, a baseband integrated circuit, an applications processor integrated circuit, a cellular network apparatus, other network apparatus, and/or other computing apparatus.

As defined herein, a "non-transitory computer readable medium," which refers to a physical medium (e.g., volatile or non-volatile memory device), can be differentiated from a "transitory computer-readable medium," which refers to an electromagnetic signal. In at least one example embodiment, a non-transitory computer readable medium is a tangible non-transitory computer readable medium.

FIG. 1 is a block diagram showing an apparatus, such as an electronic apparatus 10, according to at least one example embodiment. It should be understood, however, that an electronic apparatus as illustrated and hereinafter described is merely illustrative of an electronic apparatus that could benefit from one or more example embodiments and, therefore, should not be taken to limit the scope of the claims. While electronic apparatus 10 is illustrated and will be hereinafter described for purposes of example, other types of electronic apparatuses may readily employ one or more example embodiments. Electronic apparatus 10 may be a personal digital assistant (PDAs), a pager, a mobile computer, a desktop computer, a television, a gaming apparatus, a laptop computer, a tablet computer, a media player, a camera, a video recorder, a mobile phone, a global positioning system (GPS) apparatus, an automobile, a kiosk, an electronic table, a head mounted display, a see through display, a wearable apparatus, and/or any other types of electronic systems. Moreover, the apparatus of at least one example embodiment need not be the entire electronic apparatus, but may be a component or group of components of the electronic apparatus in other example embodiments. For example, the apparatus may be an integrated circuit, a set of integrated circuits, and/or the like.

Furthermore, apparatuses may readily employ one or more example embodiments regardless of any intent to provide mobility. In this regard, even though some example embodiments may be described in conjunction with mobile applications, it should be understood that such example embodiments may be utilized in conjunction with a variety of other applications, both in the mobile communications industries and outside of the mobile communications industries. For example, the apparatus may be, at least part of, a non-carryable apparatus, such as a large screen television, an electronic table, a kiosk, an automobile, and/or the like.

In at least one example embodiment, electronic apparatus 10 comprises at least one processor, such as processor 11 and at least one memory, such as memory 12. Processor 11 may be any type of processor, controller, embedded controller, processor core, and/or the like. In at least one example embodiment, processor 11 utilizes computer program code to cause an apparatus to perform one or more actions. Memory 12 may comprise volatile memory, such as volatile Random Access Memory (RAM) including a cache area for the temporary storage of data and/or other memory, for example, non-volatile memory, which may be embedded and/or may be removable. The non-volatile memory may comprise an EEPROM, flash memory and/or the like. Memory 12 may store any of a number of pieces of information, an amount of data, and/or the like. The information and data may be used by the electronic apparatus 10 to implement one or more functions of the electronic apparatus 10, such as the functions described herein. In at least one example embodiment, memory 12 includes computer program code such that the memory and the computer program code are configured to, working with the processor, cause the apparatus to perform one or more actions described herein.

The electronic apparatus 10 may further comprise a communication device 15. In at least one example embodiment, communication device 15 comprises an antenna, (or multiple antennae), a wired connector, and/or the like in operable communication with a transmitter and/or a receiver. In at least one example embodiment, processor 11 provides signals to a transmitter and/or receives signals from a receiver. The signals may comprise signaling information in accordance with a communications interface standard, user speech, received data, user generated data, and/or the like. Communication device 15 may operate with one or more air interface standards, communication protocols, modulation types, and access types (e.g., one or more standards in the Institute of Electrical and Electronics Engineers (IEEE) 802 family of wired and wireless standards). By way of illustration, the electronic communication device 15 may operate in accordance with second-generation (2G) wireless communication protocols IS-136 (time division multiple access (TDMA)), Global System for Mobile communications (GSM), and IS-95 (code division multiple access (CDMA)), with third-generation (3G) wireless communication protocols, such as Universal Mobile Telecommunications System (UMTS), CDMA2000, wideband CDMA (WCDMA) and time division-synchronous CDMA (TD-SCDMA), and/or with fourth-generation (4G) wireless communication protocols, wireless networking protocols, such as 802.11, short-range wireless protocols, such as Bluetooth, and/or the like. Communication device 15 may operate in accordance with wireline protocols, such as Ethernet, digital subscriber line (DSL), asynchronous transfer mode (ATM), and/or the like.

Processor 11 may comprise means, such as circuitry, for implementing audio, video, communication, navigation, logic functions, and/or the like, as well as for implementing one or more example embodiments including, for example, one or more of the functions described herein. For example, processor 11 may comprise means, such as a digital signal processor device, a microprocessor device, an analog to digital converter, a digital to analog converter, processing circuitry and other circuits, for performing various functions including, for example, one or more of the functions described herein. The apparatus may perform control and signal processing functions of the electronic apparatus 10 among these devices according to their respective capabilities. The processor 11 thus may comprise the functionality to encode and interleave message and data prior to modulation and transmission. The processor 1 may additionally comprise an internal voice coder, and may comprise an internal data modem. Further, the processor 11 may comprise functionality to operate one or more software programs, which may be stored in memory and which may, among other things, cause the processor 11 to implement at least one embodiment including, for example, one or more of the functions described herein. For example, the processor 11 may operate a connectivity program, such as a conventional internet browser. The connectivity program may allow the electronic apparatus 10 to transmit and receive internet content, such as location-based content and/or other web page content, according to a Transmission Control Protocol (TCP), Internet Protocol (IP), User Datagram Protocol (UDP), Internet Message Access Protocol (IMAP), Post Office Protocol (POP), Simple Mail Transfer Protocol (SMTP), Wireless Application Protocol (WAP), Hypertext Transfer Protocol (HTTP), and/or the like, for example.

The electronic apparatus 10 may comprise a user interface for providing output and/or receiving input. The electronic apparatus 10 may comprise an output device 14. Output device 14 may comprise an audio output device, such as a ringer, an earphone, a speaker, and/or the like. Output device 14 may comprise a tactile output device, such as a vibration transducer, an electronically deformable surface, an electronically deformable structure, and/or the like. Output device 14 may comprise a visual output device, such as a display, a light, and/or the like. In at least one example embodiment, the apparatus causes display of information, the causation of display may comprise displaying the information on a display comprised by the apparatus, sending the information to a separate apparatus, and/or the like. For example, the apparatus may send the information to a separate display, to a computer, to a laptop, to a mobile apparatus, and/or the like. For example, the apparatus may be a server that causes display of the information by way of sending the information to a client apparatus that displays the information. In this manner, causation of display of the information may comprise sending one or more messages to the separate apparatus that comprise the information, streaming the information to the separate apparatus, and/or the like. The electronic apparatus may comprise an input device 13. Input device 13 may comprise a light sensor, a proximity sensor, a microphone, a touch sensor, a force sensor, a button, a keypad, a motion sensor, a magnetic field sensor, a camera, and/or the like. A touch sensor and a display may be characterized as a touch display. In an embodiment comprising a touch display, the touch display may be configured to receive input from a single point of contact, multiple points of contact, and/or the like. In such an embodiment, the touch display and/or the processor may determine input based, at least in part, on position, motion, speed, contact area, and/or the like. In at least one example embodiment, the apparatus receives an indication of an input. The apparatus may receive the indication from a sensor, a driver, a separate apparatus, and/or the like. The information indicative of the input may comprise information that conveys information indicative of the input, indicative of an aspect of the input indicative of occurrence of the input, and/or the like.

The electronic apparatus 10 may include any of a variety of touch displays including those that are configured to enable touch recognition by any of resistive, capacitive, infrared, strain gauge, surface wave, optical imaging, dispersive signal technology, acoustic pulse recognition, or other techniques, and to then provide signals indicative of the location and other parameters associated with the touch. Additionally, the touch display may be configured to receive an indication of an input in the form of a touch event which may be defined as an actual physical contact between a selection object (e.g., a finger, stylus, pen, pencil, or other pointing device) and the touch display. Alternatively, a touch event may be defined as bringing the selection object in proximity to the touch display, hovering over a displayed object or approaching an object within a predefined distance, even though physical contact is not made with the touch display. As such, a touch input may comprise any input that is detected by a touch display including touch events that involve actual physical contact and touch events that do not involve physical contact but that are otherwise detected by the touch display, such as a result of the proximity of the selection object to the touch display. A touch display may be capable of receiving information associated with force applied to the touch screen in relation to the touch input. For example, the touch screen may differentiate between a heavy press touch input and a light press touch input. In at least one example embodiment, a display may display two-dimensional information, three-dimensional information and/or the like.

In example embodiments including a keypad, the keypad may comprise numeric (for example, 0-9) keys, symbol keys (for example, #, *), alphabetic keys, and/or the like for operating the electronic apparatus 10. For example, the keypad may comprise a conventional QWERTY keypad arrangement. The keypad may also comprise various soft keys with associated functions. In addition, or alternatively, the electronic apparatus 10 may comprise an interface device such as a joystick or other user input interface.

Input device 13 may comprise a media capturing element. The media capturing element may be any means for capturing an image, video, and/or audio for storage, display, or transmission. For example, in at least one example embodiment in which the media capturing element is a camera module, the camera module may comprise a digital camera which may form a digital image file from a captured image. As such, the camera module may comprise hardware, such as a lens or other optical component(s), and/or software for creating a digital image file from a captured image. Alternatively, the camera module may comprise only the hardware for viewing an image, while a memory device of the electronic apparatus 10 stores instructions for execution by the processor 11 in the form of software for creating a digital image file from a captured image. In at least one example embodiment, the camera module may further comprise a processing element that is separate from processor 11 for processing data, such as image data. The camera module may provide data, such as image data, in one or more of various formats. In at least one example embodiment, the camera module comprises an encoder, a decoder, and/or the like for compressing and/or decompressing image data. The encoder and/or decoder may encode and/or decode according to a standard format, for example, a Joint Photographic Experts Group (JPEG) standard format.

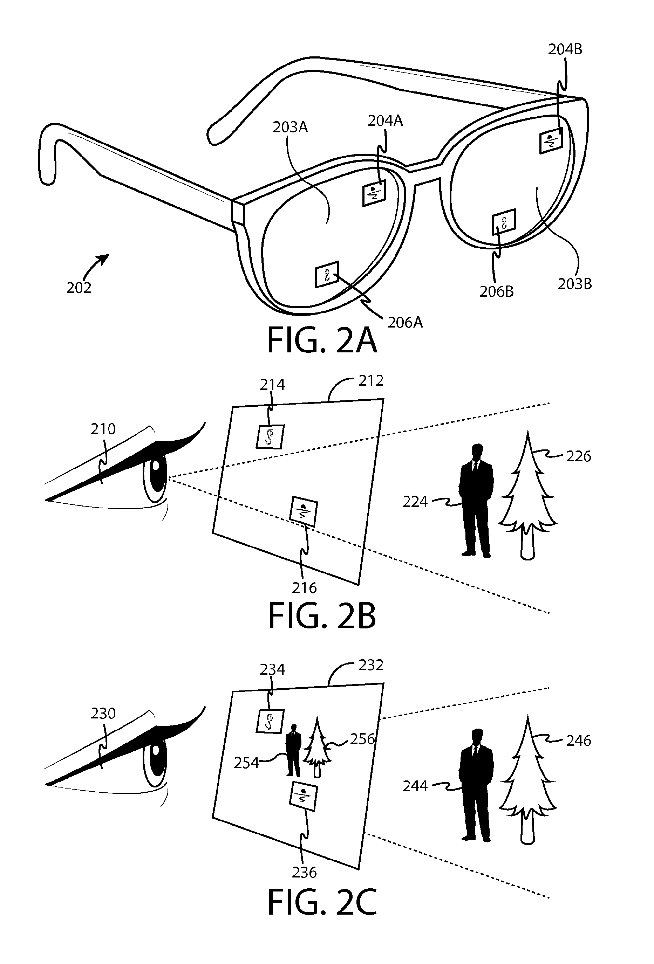

FIGS. 2A-2C are diagrams illustrating head mounted displays according to at least one example embodiment. The examples of FIGS. 2A-2C are merely examples and do not limit the scope of the claims. For example, the display type may vary, the configuration of the display may vary, the shape of the display may vary, and/or the like.

In many circumstances, a user may desire to interact with an electronic device. In such circumstances, it may often be desirable for the user to interact with an electronic apparatus by way of a head mounted display. For example, the user may interact with a program interaction screen associated with a program. In some circumstances, it may be desirable for a head mounted display to be a see through display. In at least one example embodiment, a see through display is a display that presents information to a user, but through which objects on an opposite side of the display from the user may be seen. A see through display may be comprised by a window, a windshield, a visor, glasses, and/or the like. A head mounted display may, for example, be a display that is head mountable, a display that is coupled to an element that is wearable at a location on and/or proximate to the head of a user, a display that is wearable at a location on and/or proximate to the head of a user, and/or the like. In at least one example embodiment, a head mounted display is a see through head mounted display.

FIG. 2A is a diagram illustrating display 202 according to at least one example embodiment. In the example of FIG. 2A, display 202 is illustrated as a see through display, though display 202 may be any type of display. For example, display 202 may be a non-see through display. In at least one example embodiment, a see through display is a near eye display. A near eye display may be a see through display that is positioned proximate to an eye of the user. The example of FIG. 2A illustrates display 202 as glasses that comprise a near eye display in each lens. In the example of FIG. 2A, right near eye display 203A is displaying information 204A and 206A, and left near eye display 203B is displaying information 204B and 206B. In at least one example embodiment, information 204A may be associated with information 204B. For example, the content of information 204A may be identical to content of information 204B. In some circumstances, even though the content may be identical between 204A and 204B, position of information 204A on the right near eye display may vary from position of information 204B on the left near eye display. In this manner, the apparatus may vary position of information between the left near eye display and right near eye display to vary the parallax of the information perceived by the user. In this manner, the apparatus may vary the perceived depth of the information by the user.

In some circumstances, a display, such as display 202 of FIG. 2A, may display information with a particular illumination level, the display as a whole may have an illumination level, and/or the like. For example, the display may comprise a backlight, and the backlight may have various illumination levels in different circumstances. For example, the backlight may have a low illumination level when the ambient lighting conditions near the display are dim, and the backlight may have a high illumination level when the ambient lighting conditions near the display are bright. In at least one example embodiment, a display is illuminated.

FIG. 2B is a diagram illustrating see through display 212 according to at least one example embodiment. In at least one example embodiment, displaying information on a see through display so that the information corresponds with one or more objects viewable through the see through display is referred to as augmented reality. In the example of FIG. 2B, user 210 may perceive objects 224 and 226 through see through display 212. In at least one example embodiment, the see through display may display information to the user. For example, display 212 may display information 214 and information 216. Information 214 and information 216 may be positioned on display 212 such that the information corresponds with one or more objects viewable through see through display 212, such as object 224. In such an example, information 214 may be associated with, identify, and/or the like, object 224. For example, information 214 may indicate an identity of object 224. In at least one example embodiment, display 212 may be comprised by a head mounted display.

FIG. 2C is a diagram illustrating display 232 according to at least one example embodiment. In at least one example embodiment, displaying information on a display so that the information corresponds with one or more representations of objects displayed on the display is referred to as augmented reality. In some circumstances, a representation of an object may refer to an image of an object. For example, a camera module may capture camera information that comprises information indicative of the object. The camera information may comprise video information, image information, and/or the like. This camera information may then be displayed on a display such that the camera information is a representation of the object. In the example of FIG. 2C, user 230 may perceive representations 254 and 256 displayed on display 232. Representations 254 and 256 may be images of objects captured by an apparatus. For example, a camera module may capture camera information indicative of objects 244 and 246 such that the camera information is displayed on display 232 as representations 254 and 256. In at least one example embodiment, the display may display information to the user. For example, display 232 may display information 234 and information 236. Information 234 and information 236 may be positioned on display 232 such that the information corresponds with one or more representations of objects displayed on display 232, such as representation 254. In such an example, information 234 may be associated with, identify, and/or the like, representation 254. For example, information 234 may indicate an identity of representation 254. In at least one example embodiment, display 232 may be comprised by a head mounted display.

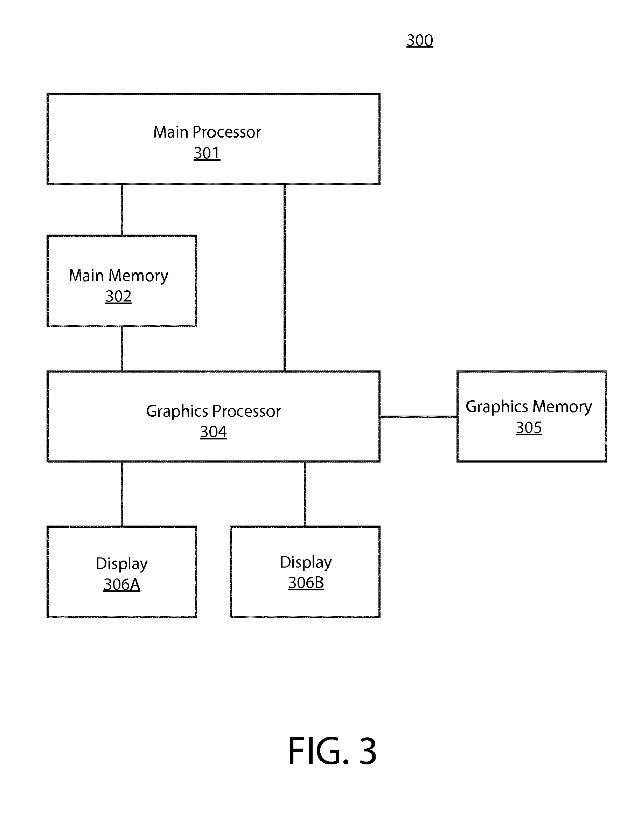

FIG. 3 is a diagram illustrating a video pipeline according to at least one example embodiment. The example of FIG. 3 is merely an example and does not limit the scope of the claims. For example, the components of the video pipeline may vary, the interconnection of components of the video pipeline may vary, the type of video pipeline may vary, and/or the like.

As previously described, a user may interact with an electronic apparatus by way of a head mounted display. For example, a user may view information displayed on the head mounted display. In some circumstances, information displayed on a head mounted display may be rendered, generated, created, and/or the like by a video pipeline. A video pipeline may refer to one more components used between an image source (e.g. a camera module, a software based rendering engine, information stored in memory, and/or the like) and an image renderer (e.g. an LCD, a CRT, an LED array, a digital micro-mirror device, and/or the like) for processing, decoding, creating, rendering, and/or the like information for display on a display. For example, a video pipeline may comprise software, a digital signal processor, a field programmable gate array (FPGA), an application specific integrated circuit (ASIC), and/or the like. In at least one example embodiment, an apparatus comprises a video pipeline. Components comprised by a video pipeline may be referred to as graphics processing hardware.

FIG. 3 is a block diagram showing video pipeline 300. Video pipeline 300 may be comprised by an apparatus, such as electronic apparatus 10 of FIG. 1, display 202 of FIG. 2A, display 212 of FIG. 2B, display 233 of FIG. 2C, and/or the like. It should be understood, however, that a video pipeline as illustrated and hereinafter described is merely illustrative of a video pipeline that could benefit from one or more example embodiments and, therefore, should not be taken to limit the scope of the claims. For example, video pipeline 300 may comprise an entire apparatus. While video pipeline 300 is illustrated and will be hereinafter described for purposes of example, other types of video pipelines may readily be employed in one or more example embodiments. Moreover, the video pipeline of at least one example embodiment need not be the entire video pipeline, but may be a component or group of components of the video pipeline in other example embodiments. For example, the video pipeline may be an integrated circuit, a set of integrated circuits, and/or the like.

In at least one example embodiment, video pipeline 300 comprises at least one main processor, such as main processor 301 and at least one main memory, such as main memory 302. Main processor 301 may be any type of processor, controller, embedded controller, processor core, and/or the like. For example, main processor 301 may be processor 11 of FIG. 1A. In at least one example embodiment, processor 301 utilizes computer program code to cause an apparatus to perform one or more actions. Main memory 302 may comprise volatile memory, such as volatile Random Access Memory (RAM) including a cache area for the temporary storage of data and/or other memory, for example, non-volatile memory, which may be embedded and/or may be removable. The non-volatile memory may comprise an EEPROM, flash memory, and/or the like. For example, main memory 302 may be memory 12 of FIG. 1A. Main memory 302 may store any of a number of pieces of information, an amount of data, and/or the like. The information and data may be used by video pipeline 300 to implement one or more functions of video pipeline 300, such as the functions described herein. In at least one example embodiment, main memory 302 includes computer program code such that the main memory and the computer program code are configured to, working with a processor, cause the apparatus to perform one or more actions described herein.

In at least one example embodiment, video pipeline 300 comprises at least one graphics processor, such as graphics processor 304 and at least one graphics memory, such as graphics memory 305. Graphics processor 305 may be any type of processor, controller, embedded controller, processor core, and/or the like. For example, main processor 301 may be processor 11 of FIG. 1A, may be a component of main processor 301, and/or the like. In at least one example embodiment, graphics processor 304 utilizes computer program code to cause an apparatus to perform one or more actions. Graphics memory 305 may comprise volatile memory, such as volatile Random Access Memory (RAM) including a cache area for the temporary storage of data and/or other memory, for example, non-volatile memory, which may be embedded and/or may be removable. The non-volatile memory may comprise an EEPROM, flash memory, and/or the like. For example, main memory 305 may be memory 12 of FIG. 1A, at least a portion of main memory 302, and/or the like. Graphics memory 305 may store any of a number of pieces of information, an amount of data, and/or the like. The information and data may be used by video pipeline 300 to implement one or more functions of video pipeline 300, such as the functions described herein. In at least one example embodiment, graphics memory 305 includes computer program code such that the main memory and the computer program code are configured to, working with a graphics processor, cause the apparatus to perform one or more actions described herein.

In at least one example embodiment, video pipeline 300 comprises one or more output devices, such as display 306A and/or display 306B. Display 306A and/or display 306B may comprise a visual output device, such as a display, a light, and/or the like. For example, display 306A and/or display 306B may comprise output device 14 of FIG. 1, display 203A and/or 203B of FIG. 2A, display 212 of FIG. 2B, display 232 of FIG. 2C, and/or the like.

In some circumstances, a head mounted display, such as display 202 of FIG. 2A, may comprise a power source that has a limited power storage capacity, such as an electrical battery, an electrical cell, a capacitor, and/or the like. For example, a head mounted display may utilize an electrical battery to power components comprised by the head mounted display, and the battery may have a particular nominal voltage, have a maximum current rating, a particular ampere-hour capacity, and/or the like. A power source with a limited power storage capacity, such as an electrical battery, may occasionally need to be recharged. For example, a head mounted display comprising an electrical battery may deplete the stored power of the electrical battery after some period of use. In some circumstances, a user may be unable to recharge a power source. For example, the user may be traveling and may have failed to travel with a charging station for the power source (e.g. a battery charger, a transformer, and/or the like), a power receptacle (e.g. an AC mains power receptacle, DC power receptacle from a vehicle, and/or the like) may be unavailable, and/or the like. In circumstances where a user may be unable to charge a power source, it may be desirable to limit consumption of the power from the power source. In other circumstances, it may be undesirable to charge a power source. For example, an available charging source may have a limited capacity, the head mounted display may be in use and charging the head mounted display may reduce the usability of the head mounted display (e.g. the head mounted display may become less mobile), and/or the like. In circumstances such as these, it may be desirable to operate the head mounted display in one or more power modes that consume less power than a different power mode of the head mounted display. In this manner, the power stored within the power source may be conserved. For example, it may be desirable to operate the head mounted display in a blink display power mode during periods when the user is not actively viewing information on the head mounted display. For example, a user may not be actively viewing information during periods when the user's eyes are closed. In another example, it may be desirable to operate the head mounted display in a non-blink display power mode during periods when the user is actively viewing information on the head mounted display. For example, a user may be actively viewing information during periods when the user's eyes are open.

A blink display power mode may refer to a power mode of the head mounted display that that consumes less power than a non-blink display power mode. For example, during a blink display power mode, one or more components of a video pipeline may be operated in a low power mode (e.g. at a lower clock speed), disabled such that the component does not draw power, and/or the like. In at least one example embodiment, power consumed by a head mounted display in a blink display power mode is less than power consumed by the head mounted display in a non-blink display power mode.

A non-blink display power mode may refer to a power mode of the head mounted display that that consumes more power than a blink display power mode. For example, during a non-blink display power mode, one or more components of a video pipeline may be operated in a non-low power mode (e.g. at a higher clock speed), enabled such that the component draws power, and/or the like. In at least one example embodiment, power consumed by a head mounted display in a non-blink display power mode is greater than power consumed by the head mounted display in a blink display power mode. As previously described, in some circumstances information may be rendered on a display. In at least one example embodiment, information is caused to be rendered on a display, comprised by a head mounted display, in a non-blink display power mode.

As previously described, in some circumstances, a head mounted display may utilize different display power modes. For example, a head mounted display may utilize one or more power modes for purposes of display of information. As previously described, a particular display power mode, such as a blink display power mode, may consume less power than a different display power mode, such as a non-blink display power mode. In at least one example embodiment, power consumed by a head mounted display for purposes of display of information in a non-blink display power mode is greater than power consumed by the head mounted display for purposes of display of information in a blink display power mode.

In some circumstances, power consumed by a head mounted display may vary based, at least in part, on the illumination of a display comprised by the head mounted display. For example, a backlight comprised by the display may draw more power when the illumination level is high, and may draw less power when the illumination level is low. For example, when the illumination level is high, the display may draw more current, operate at a higher voltage, operate at a higher wattage, consume more ampere-hours of electric charge over a period of time, and/or the like than when the illumination level is low. In circumstances such as these, it may be desirable to vary the illumination level of one more displays comprised by the head mounted display based, at least in part, on the power mode of the head mounted display. For example, it may be desirable for the head mounted display to operate with a low illumination level during a blink display power mode. In this manner, the head mounted display may draw less power during the blink display power mode. In at least one example embodiment, illumination of a display in a non-blink display power mode is greater than illumination of the display in a blink display power mode.

In some circumstances, power consumed by a head mounted display may vary based, at least in part, on whether a display comprised head mounted display is illuminated. For example, a backlight comprising the display may draw more power when the display is illuminated, and may draw little or no power when the display fails to be illuminated. In circumstances such as these, it may be desirable to terminate illumination of the head mounted display based, at least in part, on the power mode of the head mounted display. For example, it may be desirable for the head mounted display to terminate illumination level during a blink display power mode. In this manner, the head mounted display may draw less power during the blink display power mode. In at least one example embodiment, reduction of illumination of a display comprises termination of illumination of the display.

In some circumstances, it may be desirable to increase illumination of a display. For example, information displayed by the display may be difficult to read in circumstances where the illumination level is low and the ambient light level is high. Increasing the illumination of the display may facilitate viewing of information viewed on the display in these circumstances. In some circumstances, a display may fail to be illuminated. For example, as previously described, illumination of a display may have been reduced by terminating illumination of the display. In circumstances such as these, increase of illumination of the display may require initiation of illumination of the display. For example, a backlight comprised by the display may need to be turned on to illuminate the display. In at least one example embodiment, increase of illumination of a display comprises initiation of illumination of the display.

In some circumstances, power consumed by a head mounted display may vary based, at least in part, on the amount of information displayed by a display comprised by the head mounted display. For example, the display may draw more power when the displaying a large amount of information, displaying changing information, and/or the like, and may draw less power when the display is displaying little or no information, static information, and/or the like. In at least one example embodiment, information rendered by a display in a non-blink display power mode is greater than information rendered by the display in a blink display power mode. In circumstances such as these, it may be desirable to vary the amount and/or type of information rendered on a head mounted display based, at least in part, on the power mode of the head mounted display. For example, it may be desirable for the head mounted display to render less information during a blink display power mode. In another example, it may be desirable for the head mounted display to render static information during a blink display power mode. In this manner, the head mounted display may draw less power during the blink display power mode.

In some circumstances, power consumed by a head mounted display may vary based, at least in part, on whether a display is enabled or disabled. For example, the display may draw more power when the display is enabled, and may draw little or no power when the display is disabled. In circumstances such as these, it may be desirable to enable or disable the display based, at least in part, on the power mode of the head mounted display. For example, it may be desirable for the head mounted display to disable the display during a blink display power mode. In this manner, the head mounted display may draw less power during the blink display power mode. In at least one example embodiment, reduction of information rendered by a display comprises disablement of the display.

In some circumstances, it may be desirable to increase the amount of information rendered on a display. For example, a user may wish to view information on the display, view additional information on a display that is currently displaying information, and/or the like. In some circumstances, a display may fail to be enabled. For example, as previously described, a display may have been disabled. In circumstances such as these, increasing the amount of information rendered may require enablement of the display. For example, the display may need to be turned on to render information on the display. In at least one example increase of information rendered by a display comprises enablement of the display.

In some circumstances, power consumed by a head mounted display may vary based, at least in part, on power consumed by graphics processing hardware comprised by the head mounted display. For example, graphics processing hardware comprised by the head mounted display may draw may draw less power when the graphics processing hardware is operating in a low power mode. A low power mode may refer to a power mode of the graphics hardware in which the graphics hardware consumes less power. For example, the graphics hardware may operate in a low power mode by way of reducing the clock speed of the graphics hardware, disabling features of the graphics hardware, and/or the like such that the graphics hardware consumes less power. In circumstances such as these, it may be desirable to enable a low power mode of the graphics processing hardware based, at least in part, on the power mode of the head mounted display. For example, it may be desirable for graphics hardware to operate with in a low power mode during a blink display power mode of the head mounted display. In this manner, the head mounted display may draw less power during the blink display power mode. In at least one example embodiment, reduction of power consumed by graphics processing hardware comprises enablement of a low power mode of the graphics processing hardware.

In some circumstances, power consumed by a head mounted display may vary based, at least in part, on whether graphics processing hardware is enabled or disabled. For example, the display may draw more power when the graphics processing hardware is enabled, and may draw little or no power when the graphics processing hardware is disabled. In circumstances such as these, it may be desirable to enable or disable the display based, at least in part, on the power mode of the head mounted display. For example, it may be desirable for the head mounted display to disable the graphics processing hardware during a blink display power mode. In this manner, the head mounted display may draw less power during the blink display power mode. In at least one example embodiment, reduction of power consumed by graphics processing hardware comprises disablement of the graphics processing hardware.

In some circumstances, it may be desirable to disable a low power mode of the graphics processing hardware. For example, rendering information on the display may require features of the graphics processing hardware that are disabled when the lower power mode of the graphics processing hardware is enabled. Disabling the low power mode of the graphics processing hardware may facilitate rendering information on the display in these circumstances. Disabling a low power mode graphics processing hardware may cause an increase of power consumed by the graphics hardware. For example, disabling a lower power mode of graphics hardware may cause the graphics hardware to operate at a higher clock rate, enable additional features of the graphics hardware, and/or the like. In at least one example embodiment, causing an increase of power consumed by graphics processing hardware comprises disablement of a low power mode of the graphics processing hardware. For example, the apparatus may disable a previously enabled low power mode of the graphics processing hardware.

As previously described, in some circumstances, it may be desirable to increase the amount of information rendered on a display. For example, a user may wish to view information on the display, view additional information on a display that is currently displaying information, and/or the like. In some circumstances, graphics processing hardware may fail to be enabled. For example, as previously described, graphics processing hardware may have been disabled. In circumstances such as these, increasing the amount of information rendered may require enablement of graphics processing hardware. For example, the graphics processing hardware may be required to render information on the display. Enabling graphics processing hardware may cause an increase of power consumed by the graphics hardware. For example, enabling the graphics hardware may cause the graphics hardware to perform operations that consume power. In at least one example embodiment, causing an increase of power consumed by graphics processing hardware comprises enablement of the graphics processing hardware. For example, the apparatus may enable previously disabled graphics processing hardware. As previously described, a particular display power mode, such as a blink display power mode, may consume less power than a different display power mode, such as a non-blink display power mode. In at least one example embodiment, power consumed by graphics processing hardware in the non-blink display power mode is greater than power consumed by graphics processing hardware in the blink display power mode.

In some circumstances, a head mounted display may comprise non-video hardware. Non-video hardware may refer to hardware that is utilized by a head mounted display, at least in part, for purposes other than rendering information on a display. For example, non-video hardware may be used for receiving information from a server, receiving input from a user, and/or the like. It should be understood that even though non-video hardware may be utilized for purposes other than rendering information on a display, in some circumstances, non-video hardware may be utilized for rendering information on a display. For example, a processor may be utilized by a video pipeline while also being utilized as non-video hardware. For example, main processor 301 of FIG. 3 may be non-video hardware. In some circumstances, it may be desirable for non-video hardware to consume power irrespective of the display power mode. For example, it may be desirable to preclude non-video hardware from entering a low power mode in circumstances where entering a low power mode may make the non-video hardware unresponsive to inputs, slow to perform operations, and/or the like. In at least one example embodiment, power consumed by non-video hardware in a non-blink display power mode is equivalent to power consumed by non-video hardware in a blink display power mode. For example, an apparatus may avoid changing the power consumed by non-video hardware by refraining from changing settings of the non-video hardware, precluding termination of operations of the non-video hardware, and/or the like when transitioning between a display power mode and a different display power mode.

As previously described, in some circumstances, a head mounted display may operate on battery power. In such circumstances, a battery may have a particular power level. For example, battery may have a descriptive power level indicative of its state of charge, such as "high," "full," 'low," 'empty," and/or the like. In another example, a battery may have a discrete power level such as a measured voltage, a measured amount of ampere-hours remaining, a percentage of battery capacity remaining, and/or the like. A descriptive power level and a discrete power level may correspond. For example, if a battery has a fully charged capacity of 2200 mAh, a low power level may be indicative of a particular percentage of 2200 mAh remaining (e.g. 20%) in the battery. In another example, a high power level may indicated a different percentage higher than the low power percentage (e.g. 80%) is remaining in the battery. In some circumstances, it may be desirable to determine the battery power level of the head mounted display. For example, in circumstances where the battery power level is low, it may be desirable for the display to enable a low power consumption mode, a blink display power mode, a low power mode of the graphics processing hardware, and/or the like. Battery power level may be determined, for example, by measuring the voltage of the battery, monitoring the ampere-hours consumed by the battery, and/or the like. In at least one example embodiment, a battery power level of the head mounted display is determined.

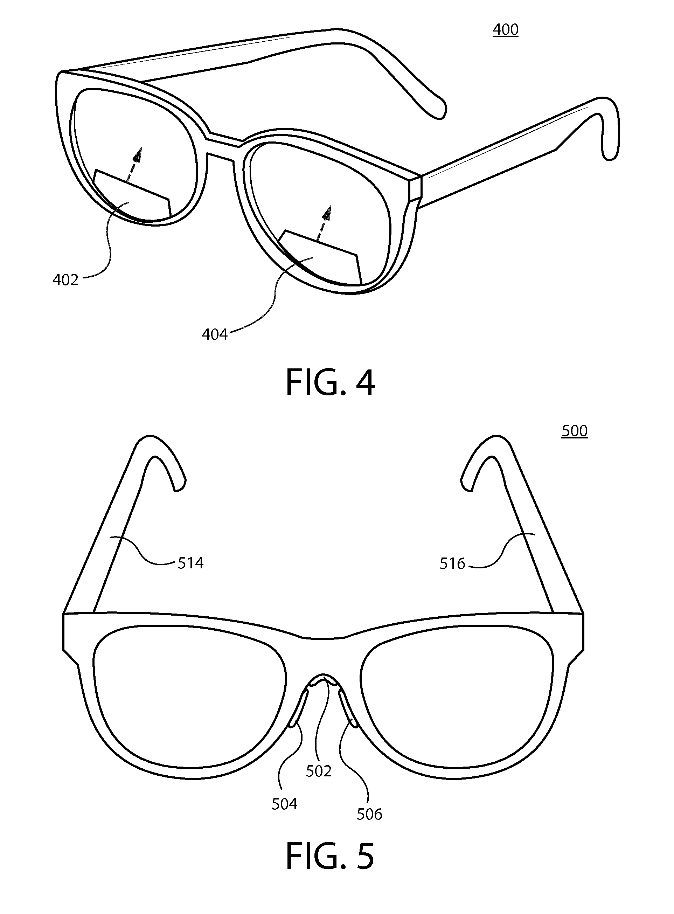

As previously described, it may be desirable to operate a head mounted display in a blink display power mode during periods when the user is not actively viewing information on the head mounted display. For example, if the user of the display is blinking, it may be desirable to operate the head mounted display in a blink display power mode during the portion of the user's blink in which the user may be unable to perceive information rendered by the head mounted display. In circumstances such as these, it may be desirable to detect eye activity of the user. Eye activity of the user may refer to the user opening one or both eyes, closing one or both eyes, blinking one or both eyes, and/or the like. For example, it may be desirable to transition from non-blink display power mode to a blink display power mode, in response to determining the user has initiated a blink. A user blink initiation may refer to a user beginning to transition from an open eye to a closed eye, similar as described regarding FIG. 4, FIG. 5, and FIG. 6. For example, an apparatus may determine a user blink initiation based on ocular sensor information received from an ocular sensor, similar as described regarding FIG. 4, FIG. 5, and FIG. 6. In at least one example embodiment, an apparatus transitions from a non-blink display power mode to a blink display power mode in response to a determination of a user blink initiation.

As previously described, in some circumstances, illumination of a display in a non-blink display power mode may be greater than illumination of the display in a blink display power mode. In at least one example embodiment, transitioning from a non-blink display power mode to a blink display power mode comprises reduction of illumination of a display. For example, transitioning from a non-blink display power mode to a blink display power mode may comprise reduction of illumination of the display, termination of illumination of the display, and/or the like.

As previously described, in some circumstances, information rendered by a display in a non-blink display power mode may be greater than information rendered by the display in a blink display power mode. In at least one example embodiment, transitioning from a non-blink display power mode to a blink display power mode comprises reduction of information rendered by a display. For example, transitioning from a non-blink display power mode to a blink display power mode may comprise disablement of the display, termination of rendering at least some information on the display, rendering the information on the display as static information, and/or the like.

As previously described, in some circumstances, power consumed by graphics processing hardware may be reduced, for example, by entering a low power mode of the graphics hardware. In at least one example embodiment, transitioning from a non-blink display power mode to a blink display power mode comprises reduction of power consumed by graphics processing hardware. For example, transitioning from a non-blink display power mode to a blink display power mode may comprise enablement of a low power mode of the graphics processing hardware, disablement of one or more features of the graphics processing hardware, and/or the like.

As previously described, in some circumstances, power consumed by non-video hardware in a non-blink display power mode may be equivalent to power consumed by non-video hardware in a blink display power mode. In at least one example embodiment, transitioning from a non-blink display power mode to a blink display power mode avoids change of power consumed by non-video hardware. For example, transitioning from a non-blink display power mode to a blink display power mode may preclude enablement of a low power mode of the non-video hardware, preclude disablement of features of the non-video hardware, and/or the like.

As previously described, it may be desirable to operate a head mounted display in a non-blink display power mode during periods when the user is actively viewing information on the head mounted display. For example, if the user of the display is not blinking, it may be desirable to operate the head mounted display in a non-blink display power mode. In circumstances such as these, it may be desirable to detect eye activity of the user. For example, it may be desirable to transition from blink display power mode to a non-blink display power mode, in response to determining the user has terminated a blink. A user blink termination may refer to a user beginning to transition from a closed eye to an open eye, similar as described regarding FIG. 4, FIG. 5, and FIG. 6. For example, an apparatus may determine a user blink termination based on ocular sensor information received from an ocular sensor, similar as described regarding FIG. 4, FIG. 5, and FIG. 6. In at least one example embodiment, an apparatus transitions from a blink display power mode to a non-blink display power mode in response to a determination of a user blink termination.

As previously described, in some circumstances, illumination of a display in a non-blink display power mode may be greater than illumination of the display in a blink display power mode. In at least one example embodiment, transitioning from a blink display power mode to a non-blink display power mode comprises increase of illumination of a display. For example, transitioning from a blink display power mode to a non-blink display power mode may comprise initiation of illumination of the display, increase of illumination of the display, and/or the like.

As previously described, in some circumstances, information rendered by a display in a non-blink display power mode may be greater than information rendered by the display in a blink display power mode. In at least one example embodiment, transitioning from a blink display power mode to a non-blink display power mode comprises increase of information rendered by a display. For example, transitioning from a blink display power mode to a non-blink display power mode may comprise enablement of the display, increasing the amount of information displayed on the display, rendering the information on a display as dynamic information, and/or the like.

As previously described, in some circumstances, power consumed by graphics processing hardware may be increased, for example, by disabling a low power mode of the graphics hardware. In at least one example embodiment, transitioning from a blink display power mode to a non-blink display power mode comprises increase of power consumed by graphics processing hardware. For example, transitioning from a blink display power mode to a non-blink display power mode may comprise disablement of a low power mode of the graphics processing hardware, enablement of features of the graphics processing hardware, and/or the like.

As previously described, in some circumstances, power consumed by non-video hardware in a non-blink display power mode may be equivalent to power consumed by non-video hardware in a blink display power mode. In at least one example embodiment, transitioning from a blink display power mode to a non-blink display power mode avoids change of power consumed by non-video hardware. For example, transitioning from a blink display power mode to a non-blink display power mode may preclude disablement of a low power mode of the non-video hardware, preclude enablement of features of the non-video hardware, and/or the like.

FIG. 4 is a diagram illustrating ocular sensors according to at least one example embodiment. The example of FIG. 4 is merely an example and does not limit the scope of the claims. For example, the number of ocular sensors may vary, the type of ocular sensors may vary, the location of the ocular sensors on the apparatus may vary, and/or the like.

As previously described, an apparatus may transition from a non-blink display power mode to a blink display power mode. For example, the apparatus may transition from a non-blink display power mode to a blink display power mode in response to a determination of a user blink initiation. In circumstances such as these, it may be desirable to determine a user blink initiation by way of ocular sensor information received from an ocular sensor. In at least one example embodiment, an apparatus determines a user blink initiation of a user based, at least in part, on ocular sensor information. An ocular sensor may refer to a sensor that may detect eye activity of the user. For example, eye activity such as blinking may be detected by a camera module, an optical sensor, a plurality of electrodes, and/or the like. In at least one example embodiment, an apparatus receives ocular sensor information from at least one ocular sensor. Ocular sensor information may refer to information received from, transmitted by, and/or the like from an ocular sensor indicative of eye activity. For example, ocular sensor information may indicate that a user has initiated a blink, terminated a blink, has open eyes, has closed eyes, and/or the like. In at least one example embodiment, ocular sensor information indicates eye activity of a user of a head mounted display.

Similarly, as previously described, in some circumstances an apparatus may transition from a blink display power mode to a non-blink display power mode. For example, the apparatus may transition from a blink display power mode to a non-blink display power mode in response to a determination of a user blink termination. In circumstances such as these, it may be desirable to determine a user blink termination by way of ocular sensor information received from an ocular sensor. For example, an apparatus may receive ocular sensor information indicative of a user blink initiation. In response, the apparatus may determine a user blink termination based, at least in part, on an average blink duration, receipt of different ocular sensor information indicative of a user blink termination, and/or the like. In at least one example embodiment, an apparatus determines a user blink termination of a user based, at least in part, on ocular sensor information;

FIG. 4 is a diagram illustrating head mounted display 400 according to at least one example embodiment. In the example of FIG. 4, head mounted display 400 is illustrated as a see through display, though head mounted display 400 may be any type of display. For example, head mounted display 400 may be a non-see through display. The example of FIG. 4 illustrates head mounted display 400 as glasses that comprise a near eye display in each lens. In the example of FIG. 4, head mounted display 400 is illustrated as having ocular sensor 402 positioned near the right lens of head mounted display 400, and ocular sensor 404 positioned near the left lens of head mounted display 400. Even though the example of FIG. 4 illustrates ocular sensors 402 and 404 at particular positions relative to head mounted display 400, it should be understood that an ocular sensor may be placed at any position on a head mounted display.

As previously described, eye activity such as blinking may be detected by a camera module. For example, ocular sensors 402 and 404 of FIG. 4A may comprise camera modules. In such an example, the camera modules comprised by ocular sensors 402 and 404 may detect eye activity of a user of head mounted display 400. For example, the user of head mounted display 400 may blink. In at least one example embodiment, an ocular sensor comprises at least one camera module.

As previously described, a camera module may provide data, such as image data, in one or more of various formats. For example, the image data may comprise ocular sensor information, such as visual information indicative of an eye of a user. For example, visual information captured by a camera module may indicate that a user has an open eye, a closed eye, an eye transitioning from open to closed, an eye transitioning from closed to open, and/or the like. In at least one example embodiment, ocular sensor information comprises visual information indicative of an eye of the user.

As previously described, in some circumstances an apparatus may determine a user blink initiation. In at least one example embodiment, determination of a user blink initiation comprises determination of visual information indicative of a transition from the eye being open to the eye being closed. For example, the apparatus may receive visual information from a camera module indicative of a transition from the eye being open to the eye being closed.

As previously described, in some circumstances an apparatus may determine a user blink termination. In at least one example embodiment, determination of a user blink termination comprises determination of visual information indicative of a transition from the eye being closed to the eye being open. For example, the apparatus may receive visual information from a camera module indicative of a transition from the eye being closed to the eye being open.

As previously described, eye activity such as blinking may be detected by an optical sensor. For example, ocular sensors 402 and 404 of FIG. 4A may comprise optical sensors. An optical sensor may refer to a sensor which may detect a change in illumination, color, position, and/or the like. In such an example, the optical sensors comprised by ocular sensors 402 and 404 may detect eye activity of a user of head mounted display 400. For example, an optical sensory may distinguish the white portion of an eye from the darker surrounding skin of the eyelids. In this manner, an optical sensor may determine that an eye is open, closed, transitioning from closed to open, transitioning from open to closed, and/or the like.

As previously described, a user blink initiation, blink termination, and/or the like may be referred to as eye activity. For example, ocular sensor information may indicate eye activity as being a user blink initiation. In some circumstances, such eye activity may be followed by other eye activity. For example, the user may perform a blink termination. In circumstances where such eye activity is detected, the ocular sensor may transmit other ocular information that indicates the other eye activity. Such other eye activity may be used to determine a user blink termination. In at least one example embodiment, an apparatus receives other information indicating other eye activity of a user. In this manner, determination of a user blink termination may be based, at least in part, on the other ocular sensor information.

FIG. 5 is a diagram illustrating ocular sensors according to at least one example embodiment. The example of FIG. 5 is merely an example and does not limit the scope of the claims. For example, the number of ocular sensors may vary, the type of ocular sensors may vary, the location of the ocular sensors on the apparatus may vary, and/or the like.

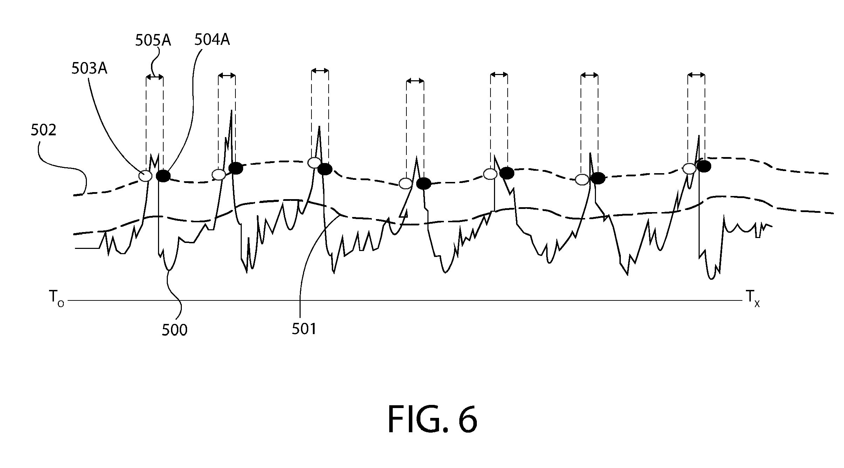

As previously described, eye activity such as blinking may be detected by a plurality of electrodes. For example, a plurality of electrodes may comprise an ocular sensor that captures electrooculography (EOG) information, similar as described regarding FIG. 6. An electrode may refer to an electrical conductor configured to electrically couple or contact skin of the user with a circuit. It should be understood that an electrode may be different from an electrical connector. Generally, an electrode may be used to make contact with a non-metallic or non-conductive portion of a circuit (e.g. a portion of body skin), whereas an electrical connector will typically join two metallic portions of a circuit (e.g. a plug and a jack). In at least one example embodiment, an ocular sensor comprises a plurality of electrodes.

FIG. 5 is a diagram illustrating head mounted display 500 according to at least one example embodiment. In the example of FIG. 5, head mounted display 500 is illustrated as a see through display, though head mounted display 500 may be any type of display. For example, head mounted display 500 may be a non-see through display. The example of FIG.5 illustrates head mounted display 500 as glasses that comprise a near eye display in each lens. In the example of FIG. 5, head mounted display 500 is illustrated as having electrode 502 disposed on a middle portion of a bridge of the glasses comprised by head mounted display 500. In this manner, electrode 502 is configured to contact a front of a user of head mounted display 500's nose bridge. In the example of FIG. 5, head mounted display 500 is illustrated as having electrode 504 disposed on the right side of the bridge of the glasses comprised by head mounted display 500. In this manner, electrode 504 is configured to contact a right side of the user's nose bridge. In the example of FIG. 5, head mounted display 500 is illustrated as having electrode 506 disposed near the left side of the bridge of the glasses comprised by head mounted display 500. In this manner, electrode 506 is configured to contact a left side of the user's nose bridge. Even though the example of FIG. 5 illustrates electrodes 502, 504, and 506 disposed at particular positions on head mounted display 500, it should be understood that an electrode may be placed at any position on a head mounted display. For example, an electrode may be placed on temple 512 and/or on temple 516 of head mounted display 500.

FIG. 6 is a diagram illustrating ocular sensor information according to at least one example embodiment. The example of FIG. 6 is merely an example and does not limit the scope of the claims. For example, the type of ocular sensor information may vary, the amount of ocular sensor information may vary, the format of the ocular sensor information may vary, and/or the like.

As previously described, an ocular sensor may comprise a plurality of electrodes. For example, an ocular sensor may comprise one or more electrodes configured to contact a nose of a user, similar as described regarding FIG. 5. In circumstances such as these, ocular sensor information may comprise EOG information indicative of an eye of the user. EOG may refer to a technique for measuring a corneo-retinal standing potential that exists between the front and the back of the human eye. For example, a plurality of electrodes may be placed near an eye, and movement of the eye, blinking of the eye, and/or the like may be detected by the plurality of electrodes. The resulting signal may be referred to as an electrooculogram, EOG information, and/or the like. For example, EOG information captured by a plurality of electrodes may indicate that a user has an open eye, a closed eye, an eye transitioning from open to closed, an eye transitioning from closed to open, and/or the like.

The example of FIG. 6 illustrates EOG information recorded from a time To to a time Tₓ. In the example of FIG. 6, the EOG information comprises a vertical component 500 that varies over the time period between time To and time Tₓ. Vertical component 500 may be interpreted as a series of discrete data points that correspond with particular times. For example, it can be seen that data point 503A of FIG. 6 corresponds with a time occurring before a time corresponding with data point 504A. The time between individual data points may be described as a duration. For example, a duration may be measured between data points 503A and 504A as duration 505A.

Eye blinks may be detected by analyzing the vertical component of the EOG information. For example, an apparatus may detect a blink initiation whenever the vertical EOG component increases to exceed predetermined maximum value. Such a predetermined value may be referred to as a blink threshold value. For example, dashed line 502 of FIG. 6 may represent a predetermined maximum value. In this manner, dashed line 502 of FIG. 6 may represent a blink threshold value. It can be seen in the example of FIG. 6 that vertical component 500 has increased to exceed the value of dashed line 502 at a time corresponding with data point 503A of FIG. 6. In this manner, vertical component 500 may indicate a blink initiation at the time corresponding with data point 503A of FIG. 6.

As previously described, in some circumstances an apparatus may determine a user blink initiation. In at least one example embodiment, determination of a user blink initiation comprises determination that EOG information indicates a transition from the eye being open to the eye being closed. For example, EOG information may indicate that a vertical component of the EOG information has increased to exceed a blink threshold value. In this manner, the EOG information may indicate a transition from the eye being open to the eye being closed. In at least one example embodiment, the blink threshold value is a predetermined value. For example, the blink threshold value may be a constant, a value stored in memory, a previously established user preference, and/or the like.

In some circumstances, an EOG signal from a plurality of electrodes may be noisy. For example, the electrodes may receive electromagnetic interference, the position of the electrodes on a user may be unstable, and/or the like. In such circumstances, blink detection may be inaccurate if the blink threshold value is a stored value. For example, noise may cause a false indication of a user blink if the noise is above the blink threshold value. In circumstances such as these, it may be desirable to calculate a running average of the vertical component of the EOG information as a blink threshold. The running average of the vertical component of EOG information may be calculated as a running N-point average of the vertical component up to time t. N may be, for example, equal to the number of samples corresponding to 20ms, 100ms or 500ms, or any other suitable value. The blink threshold value may be represented as proportional to the N-point average value. For example, the detection threshold may be defined to be equal to two times the N-point average value. For instance, in the example of FIG. 6, dashed line 501 may represent the running average of vertical component 500, and dashed line 502 may represent two times the running average of vertical component 500. It can be seen in the example of FIG. 6 that vertical component 500 has increased to exceed the value of dashed line 502 at a time corresponding with data point 503A of FIG. 6. In this manner, vertical component 500 may indicate a blink initiation at the time corresponding with data point 503A of FIG. 6. In at least one example embodiment, an apparatus determines a running average of EOG information over a predetermined duration, and determines a blink threshold value to be proportional to the running average.

In another example, the blink threshold may be defined to be proportional to the standard deviation of the vertical component of the EOG information. For example, the blink threshold may be defined to equal the level N point average at time t + 2 times the standard deviation of the EOG signal. For instance, in the example of FIG. 6, dashed line 501 may represent the running average of vertical component 500, and dashed line 502 may represent the sum of the running average 501 and two times the standard deviation of vertical component 500. It can be seen in the example of FIG. 6 that vertical component 500 has increased to exceed the value of dashed line 502 at a time corresponding with data point 503A of FIG. 6. In this manner, vertical component 500 may indicate a blink initiation at the time corresponding with data point 503A of FIG. 6. In at least one example embodiment, an apparatus determines a standard deviation of EOG information over a predetermined duration. In this manner, determination of a blink threshold value may be performed such that the blink threshold value is proportional to the standard deviation.

As previously described, in some circumstances an apparatus may determine a user blink termination. For example, an apparatus may detect a blink termination whenever a vertical EOG component decreases to become within a blink threshold value. For example, dashed line 502 of FIG. 6 may represent a blink threshold value. It can be seen in the example of FIG. 6 that vertical component 500 has decreased to become within the value of dashed line 502 at a time corresponding with data point 504A of FIG. 6. In this manner, vertical component 500 may indicate a blink termination at the time corresponding with data point 504A of FIG. 6. In at least one example embodiment, determination of a user blink termination comprises determination that the EOG information indicates a transition from the eye being closed to the eye being open. For example, EOG information may indicate that a vertical component of the EOG information has decreased to become within a blink threshold value. In this manner, the EOG information may indicate a transition from the eye being closed to the eye being open.

As previously described, in some circumstances an apparatus may determine a battery power level of a head mounted display. In circumstances where the battery level is known, it may be desirable to adjust a blink threshold based, at least in part, on the battery power level. For example, it may be desirable to lower the blink threshold when the battery power is low. In this manner, a blink display power mode may trigger sooner than when the blink threshold is higher. For instance in circumstances where the blink threshold is the running N-point average + 2 times the standard deviation, a suitable blink threshold in the case of lowered battery level might be running N-point average + 1 time the standard deviation. In this manner, a blink initiation may be detected earlier and a blink termination may be detected later. In circumstances where a blink initiation and a blink termination cause the system to transition between non-blink and blink display power modes, such a lower blink threshold may cause the apparatus to transition to the blink display power mode earlier, and transition to the non-blink display power mode later. The detection threshold may be adjusted lower as the battery level decreases, and adjusted higher as the battery level increases (e.g. when the battery is charging). For example, a user may be more tolerant of noticeable changes between display power modes when the battery power is low, but may prefer that changes in display power modes are unperceivable when the battery is fully charged. In at least one example embodiment, an apparatus determines a blink threshold value to be proportional to a battery power level.

A blink may have a particular duration. For example, a blink may have a blink duration that is the period of time between a blink initiation and a blink termination. For instance, in the example of FIG. 6A, a blink having a blink initiation corresponding with the time of data point 503A and a blink termination corresponding with the time of data point 503B may have a blink duration corresponding with duration 505A. In some circumstances, it may be desirable for an apparatus to determine an average blink duration. For example, determination of a user blink termination may be based on elapse of an average blink duration subsequent to a user blink initiation. In this manner, a blink termination may be determined without determining that a vertical component of EOG information has decreased to become within a blink threshold value. A series of blink durations may be averaged over a particular period to determine an average blink duration. In at least one example embodiment, an apparatus determines a user blink termination based, at least in part, on elapse of an average blink duration subsequent to a user blink initiation.

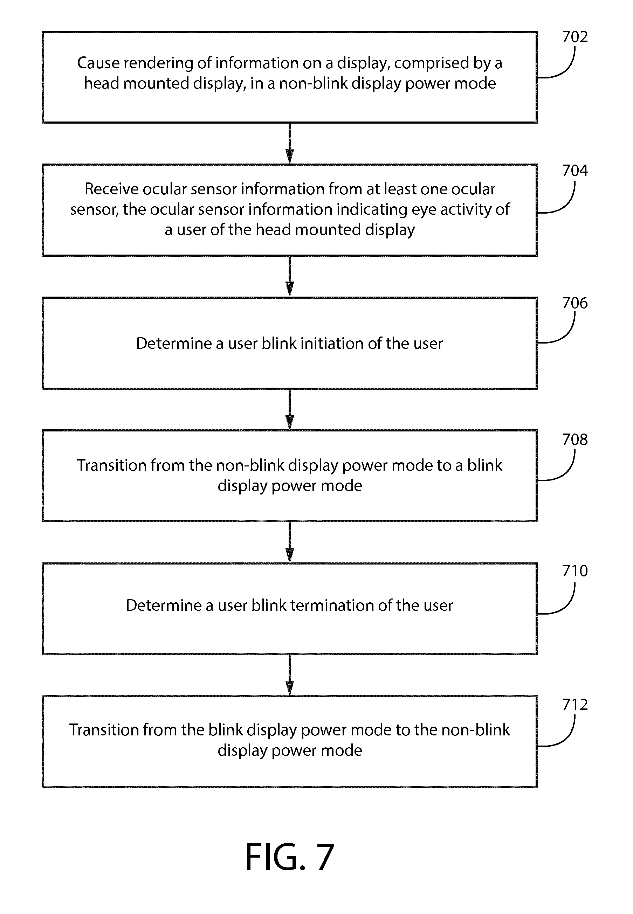

FIG. 7 is a flow diagram illustrating activities associated with transition from a display power mode to a different display power mode according to at least one example embodiment. In at least one example embodiment, there is a set of operations that corresponds with the activities of FIG. 7. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 7.

As previously described, in some circumstances, it may be desirable for an apparatus to transition from a non-blink display power mode to a blink display power mode.

At block 702, the apparatus causes rendering of information on a display, comprised by a head mounted display, in a non-blink display power mode. The rendering, the information, the display, the head mounted display, and the non-blink power mode may be similar as described regarding FIG. 1, FIGS. 2A-2C, FIG. 3, FIG. 4, FIG. 5, and FIG. 6.

At block 704, the apparatus receives ocular sensor information from at least one ocular sensor, the ocular sensor information indicating eye activity of a user of the head mounted display. The receipt, the ocular sensor information, the ocular sensor, and the eye activity may be similar as described regarding FIG. 1, FIGS. 2A-2C, FIG. 3, FIG. 4, FIG. 5, and FIG. 6.

At block 706, the apparatus determines a user blink initiation of the user based, at least in part, on the ocular sensor information. The determination and the user blink initiation may be similar as described regarding FIG. 3, FIG. 4, FIG. 5, and FIG. 6.

At block 708, in response to the determination of the user blink initiation, the apparatus transitions from the non-blink display power mode to a blink display power mode. The transitioning and the blink display power mode may be similar as described regarding FIG. 3, FIG. 4, FIG. 5, and FIG. 6.

At block 710, the apparatus determines a user blink termination of the user based, at least in part, on the ocular sensor information. The determination and the user blink termination may be similar as described regarding FIG. 3, FIG. 4, FIG. 5, and FIG. 6.

At block 712, in response to the determination of the user blink termination, the apparatus transitions from the blink display power mode to the non-blink display power mode. The transition may be similar as described regarding FIG. 3, FIG. 4, FIG. 5, and FIG. 6.

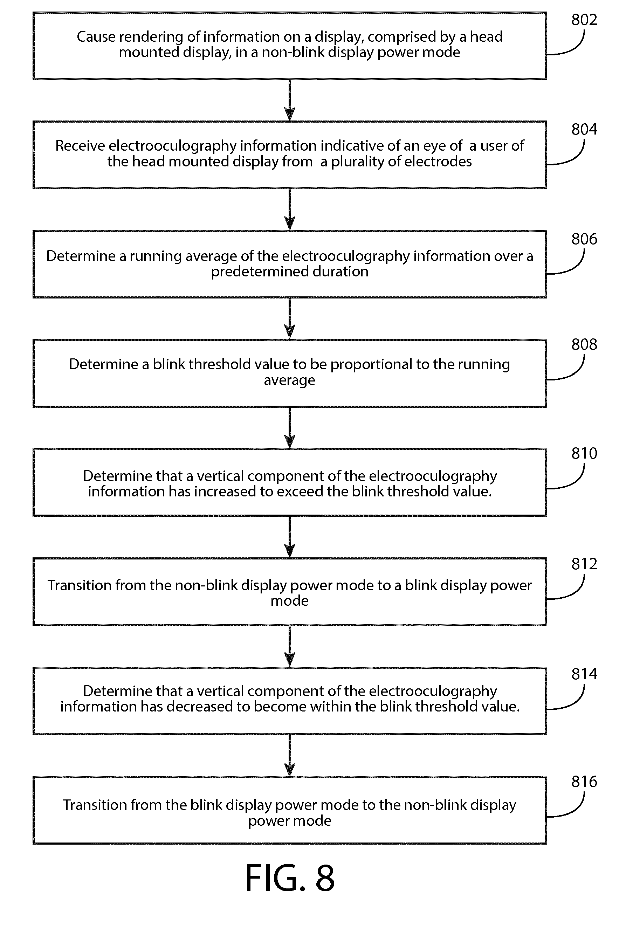

FIG. 8 is a flow diagram illustrating activities associated with receipt of electrooculography information according to at least one example embodiment. In at least one example embodiment, there is a set of operations that corresponds with the activities of FIG. 8. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 8.

As previously described, in some circumstances it may be desirable for an apparatus to receive electrooculography information.

At block 802, the apparatus causes rendering of information on a display, comprised by a head mounted display, in a non-blink display power mode, similarly as described regarding block 702 of FIG. 7. At block 804, the apparatus receives electrooculography information, indicative of an eye of a user of the head mounted display, from a plurality of electrodes. The receipt, the electrooculography information, the eye of the user, and the electrodes may be similar as described regarding FIG. 3, FIG. 5, and FIG. 6.

At block 806, the apparatus determines a running average of the electrooculography information over a predetermined duration. The determination, the running average, and the predetermined duration may be similar as described regarding FIG. 3, FIG. 5, and FIG. 6.

At block 808, the apparatus determines a blink threshold value to be proportional to the running average. The determination, the blink threshold value, and the proportionality may be similar as described regarding FIG. 3, FIG. 5, and FIG. 6.

At block 810, the apparatus determines that a vertical component of the electrooculography information has increased to exceed the blink threshold value. The determination, the vertical component, the increase, and the blink threshold value may be similar as described regarding FIG. 3, FIG. 5, and FIG. 6. At block 812, in response to the determination that the vertical component of the electrooculography information has increased to exceed the blink threshold value, the apparatus transitions from the non-blink display power mode to a blink display power mode, similarly as described regarding block 708 of FIG. 7.

At block 814, the apparatus determines that a vertical component of the electrooculography information has decreased to become within the blink threshold value. The determination, the vertical component, and the decrease may be similar as described regarding FIG. 3, FIG. 5, and FIG. 6. At block 816, in response to the determination that the vertical component of the electrooculography information has decreased to become within the blink threshold value, the apparatus transitions from the blink display power mode to the non-blink display power mode, similarly as described regarding block 712 of FIG. 7.

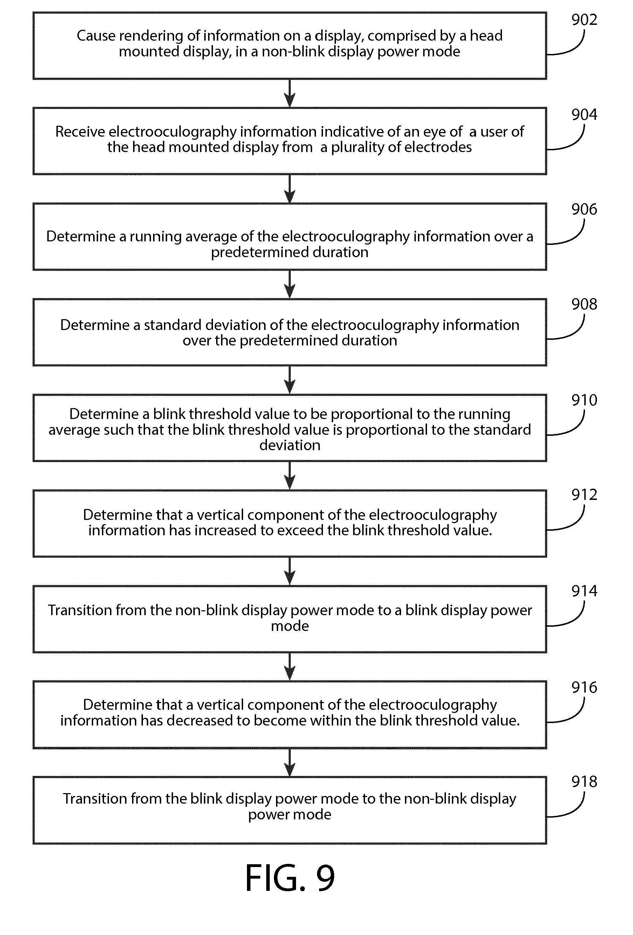

FIG. 9 is a flow diagram illustrating activities associated with determination of a standard deviation of electrooculography information according to at least one example embodiment. In at least one example embodiment, there is a set of operations that corresponds with the activities of FIG. 9. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 9.

As previously described, in some circumstances it may be desirable for an apparatus to determine a standard deviation of electrooculography information.

At block 902, the apparatus causes rendering of information on a display, comprised by a head mounted display, in a non-blink display power mode, similarly as described regarding block 702 of FIG. 7. At block 904, the apparatus receives electrooculography information, indicative of an eye of a user of the head mounted display, from a plurality of electrodes, similarly as described regarding block 804 of FIG. 8. At block 906, the apparatus determines a running average of the electrooculography information over a predetermined duration, similarly as described regarding block 806 of FIG. 8.

At block 908, the apparatus determines a standard deviation of the electrooculography information over the predetermined duration. The determination and the standard deviation may be similar as described regarding FIG. 3, FIG. 5, and FIG. 6.

At block 910, the apparatus determines a blink threshold value to be proportional to the running average such that the blink threshold value is proportional to the standard deviation. The determination, the blink threshold, and the proportionality may be similar as described regarding FIG. 3, FIG. 5, and FIG. 6.

At block 912, the apparatus determines that a vertical component of the electrooculography information has increased to exceed the blink threshold value, similarly as described regarding block 810 of FIG. 8. At block 914, in response to the determination that the vertical component of the electrooculography information has increased to exceed the blink threshold value, the apparatus transitions from the non-blink display power mode to a blink display power mode, similarly as described regarding block 708 of FIG. 7. At block 916, the apparatus determines that a vertical component of the electrooculography information has decreased to become within the blink threshold value similarly as described regarding block 814 of FIG. 8. At block 918, in response to the determination that the vertical component of the electrooculography information has decreased to become within the blink threshold value, similarly as described regarding block 816 of FIG. 8.

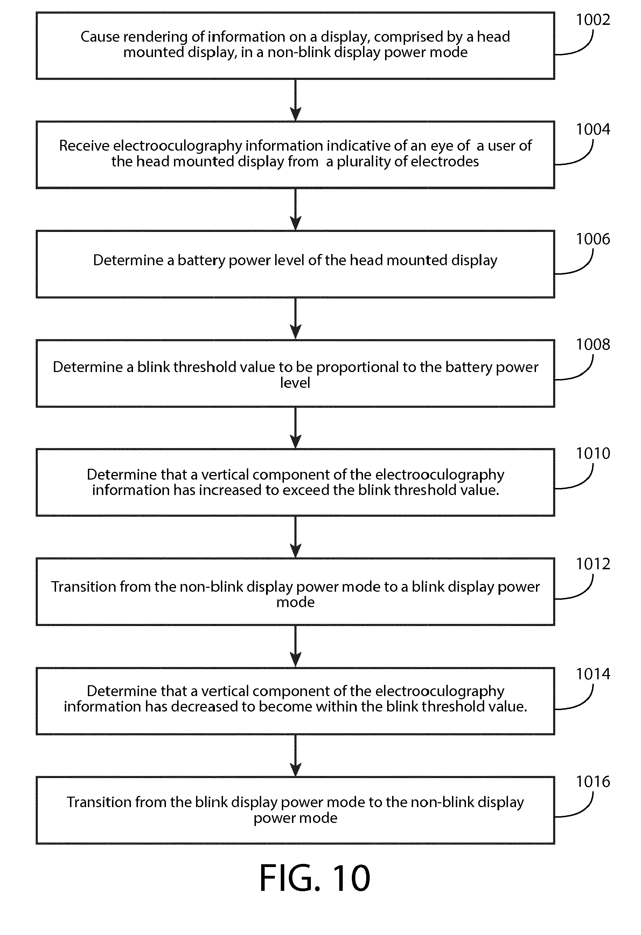

FIG. 10 is a flow diagram illustrating activities associated with determination of a battery power level according to at least one example embodiment. In at least one example embodiment, there is a set of operations that corresponds with the activities of FIG. 10. An apparatus, for example electronic apparatus 10 of FIG. 1, or a portion thereof, may utilize the set of operations. The apparatus may comprise means, including, for example processor 11 of FIG. 1, for performance of such operations. In an example embodiment, an apparatus, for example electronic apparatus 10 of FIG. 1, is transformed by having memory, for example memory 12 of FIG. 1, comprising computer code configured to, working with a processor, for example processor 11 of FIG. 1, cause the apparatus to perform set of operations of FIG. 10.

As previously described, in some circumstances it may be desirable for an apparatus to determine a battery power level.

At block 1002, the apparatus causes rendering of information on a display, comprised by a head mounted display, in a non-blink display power mode, similarly as described regarding block 702 of FIG. 7. At block 1004, the apparatus receives electrooculography information, indicative of an eye of a user of the head mounted display, from a plurality of electrodes, similarly as described regarding block 804 of FIG. 8.

At block 1006, the apparatus determines a battery power level of the head mounted display. The determination and the battery power level may be similar as described regarding FIG. 3, FIG. 5, and FIG. 6.

At block 1008, the apparatus determines a blink threshold value to be proportional to the battery power level. The determination, the blink threshold value, and the proportionality may be similar as described regarding FIG. 3, FIG. 5, and FIG. 6.

At block 1010, the apparatus determines that a vertical component of the electrooculography information has increased to exceed the blink threshold value, similarly as described regarding block 810 of FIG. 8. At block 1012, in response to the determination that the vertical component of the electrooculography information has increased to exceed the blink threshold value, the apparatus transitions from the non-blink display power mode to a blink display power mode, similarly as described regarding block 708 of FIG. 7. At block 1014, the apparatus determines that a vertical component of the electrooculography information has decreased to become within the blink threshold value similarly as described regarding block 814 of FIG. 8. At block 1016, in response to the determination that the vertical component of the electrooculography information has decreased to become within the blink threshold value, similarly as described regarding block 816 of FIG. 8.

One or more example embodiments may be implemented in software, hardware, application logic or a combination of software, hardware, and application logic. The software, application logic, and/or hardware may reside on the apparatus, a separate device, or a plurality of separate devices. If desired, part of the software, application logic, and/or hardware may reside on the apparatus, part of the software, application logic and/or hardware may reside on a separate device, and part of the software, application logic, and/or hardware may reside on a plurality of separate devices. In an example embodiment, the application logic, software or an instruction set is maintained on any one of various computer-readable media.

If desired, the different functions discussed herein may be performed in a different order and/or concurrently with each other. For example, block 702 of FIG. 7 may be performed after block 712 of FIG. 7. Furthermore, if desired, one or more of the above-described functions may be optional or may be combined. For example, block 702 of FIG. 7 may be optional and/or combined with block 712 of FIG. 7.

Although various aspects of the present subject matter are set out in the independent claims, other aspects of the present subject matter comprise other combinations of features from the described example embodiments and/or the dependent claims with the features of the independent claims, and not solely the combinations explicitly set out in the claims.

It is also noted herein that while the above describes example embodiments, these descriptions should not be viewed in a limiting sense. Rather, there are variations and modifications which may be made without departing from the scope of the present subject matter.

## Claims

1. A method comprising:
causing rendering of information on a display, comprised by a head mounted display, in a non-blink display power mode;
receiving ocular sensor information from at least one ocular sensor, the ocular sensor information indicating eye activity of a user of the head mounted display;
determining a user blink initiation of the user based, at least in part, on the ocular sensor information;
transitioning from the non-blink display power mode to a blink display power mode in response to the determination of the user blink initiation;
determining a user blink termination of the user based, at least in part, on the ocular sensor information; and
transitioning from the blink display power mode to the non-blink display power mode in response to the determination of the user blink termination.

2. The method of Claim 1, wherein the ocular sensor comprises a plurality of electrodes, and the ocular sensor information comprises electrooculography information indicative of an eye of the user.

3. The method of any of Claim 2, wherein the head mounted display is configured to have at least one of the plurality of electrodes contact a front of the user's nose bridge.

4. The method of any of Claim 2, wherein the head mounted display is configured to have at least one of the plurality of electrodes contact a right side of the user's nose bridge.

5. The method of any of Claim 2, wherein the head mounted display is configured to have at least one of the plurality of electrodes contact a left side of the user's nose bridge.

6. The method of any of Claims 2-5, wherein the determination of a user blink initiation comprises determination that the electrooculography information indicates a transition from the eye being open to the eye being closed.

7. The method of Claim 6, wherein the determination that the electrooculography information indicates a transition from the eye being open to the eye being closed comprises determination that a vertical component of the electrooculography information has increased to exceed a blink threshold value.

8. The method of Claim 7, further comprising:
Determination of a running average of the electrooculography information over a predetermined duration; and
Determination of the blink threshold value to be proportional to the running average.

9. The method of Claim 8, further comprising determination of a standard deviation of the electrooculography information over the predetermined duration, wherein the determination of the blink threshold value is performed such that the blink threshold value is proportional to the standard deviation.

10. The method of any of Claims 2-9, wherein the determination of a user blink termination comprises determination that the electrooculography information indicates a transition from the eye being closed to the eye being open.

11. The method of Claim 10, wherein the determination that the electrooculography information indicates a transition from the eye being closed to the eye being open comprises determination that a vertical component of the electrooculography information has decreased to become within a blink threshold value.

12. A method comprising:
causing rendering of information on a display, comprised by a head mounted display, in a non-blink display power mode;
receiving electrooculography information from a plurality of electrodes, the electrooculography indicative of an eye of a user of the head mounted display;
determining a running average of the electrooculography information over a predetermined duration;
determining a standard deviation of the electrooculography information over the predetermined duration;
determining a blink threshold value to be proportional to the running average, wherein the determination of the blink threshold value is performed such that the blink threshold value is proportional to the standard deviation;
determining that the electrooculography information indicates a transition from the eye being open to the eye being closed based, at least in part, on the electrooculography information, wherein the determination that the electrooculography information indicates a transition from the eye being open to the eye being closed comprises determination that a vertical component of the electrooculography information has increased to exceed the blink threshold value;
transitioning from the non-blink display power mode to a blink display power mode in response to the determination that the electrooculography information indicates a transition from the eye being open to the eye being closed;
determining that the electrooculography information indicates a transition from the eye being closed to the eye being open; and
transitioning from the blink display power mode to the non-blink display power mode in response to the determination that the electrooculography information indicates a transition from the eye being closed to the eye being open, wherein the determination that the electrooculography information indicates a transition from the eye being closed to the eye being open comprises determination that a vertical component of the electrooculography information has decreased to become within the blink threshold value.

13. An apparatus comprising means for performing the method of any of the preceding claims.

14. The apparatus of Claim 13, wherein the means for performing the method comprise at least one processor and at least one memory, the memory comprising machine-readable instructions, that when executed cause the apparatus to perform the method of any of claims 1-12.

15. At least one computer readable medium comprising instructions that, when executed, perform the method of any of claims 1-12.
